# EUROPÄISCHE PATENTANMELDUNG

(11) **EP 3 185 028 A1**
(43) Veröffentlichungstag der Anmeldung: **28.06.2017**
(21) Anmeldenummer: 16199854.7
(22) Anmeldetag: 21.11.2016
(51) Int. Cl.: G01R 33/465, G01R 33/38, G01R 33/483, G01R 33/44

(54) **VERWENDUNG EINES NMR-MESSGERÄTS ZUR UNTERSUCHUNG VON BESTANDTEILEN EINES MENSCHLICHEN ODER TIERISCHEN KÖRPERS**

(30) Priorität: 21.12.2015 DE 102015226168
(71) Anmelder: ROBERT BOSCH GMBH, 70442 Stuttgart (DE)
(72) Erfinder: Krapf, Reiner, 70794 Filderstadt (DE); Marx, Klaus, 70563 Stuttgart (DE); Graessl, Andreas, 70771 Leinfelden-Echterdingen (DE); Hoffmann, Ulli, 75223 Niefern-Oeschelbronn (DE)

(57) **Zusammenfassung**

Es wird vorgeschlagen, ein handgehaltenes, energieautonomes Messgerät (10) mit einem Gehäuse (12), in dem zumindest ein Kernspinresonanz-Sensor (32), eine Steuervorrichtung (28) zur Steuerung des Messgeräts (10), eine Auswertevorrichtung (30) zur Auswertung eines von dem Kernspinresonanz-Sensor (32) gelieferten Messsignals, eine Ausgabevorrichtung (16, 16') zur Ausgabe von ermittelten Informationen sowie eine Energieversorgungsvorrichtung (22) des Messgeräts (10) in Form einer Batterie, insbesondere einer wiederaufladbaren Batterie, vorgesehen sind, zur Untersuchung eines Bestandteils (42) eines menschlichen oder tierischen Körpers, insbesondere zur Untersuchung von Gewebe und/oder Körperflüssigkeiten, bevorzugt zur Untersuchung von Blut und/oder Urin, zu verwenden.

## Beschreibung

### Stand der Technik

Die vorliegende Erfindung betrifft die Verwendung eines mobilen, handgehaltenen Messgeräts mit einem Kernspinresonanz-Sensor.

Aus DE 10 2014 218 375 A1 und DE 10 2014 218 371 A1 sind mobile Messgeräte mit jeweils einer Sensorvorrichtung bekannt, wobei die Sensorvorrichtung zumindest einen Kernspinresonanz-Sensor aufweist, der zur Bestimmung eines Feuchtewerts bzw. zur Detektion und/oder Analyse und/oder Unterscheidung von Materialkennwerten eines zu untersuchenden Werkstücks vorgesehen ist.

### Offenbarung der Erfindung

Erfindungsgemäß wird die Verwendung eines handgehaltenen, energieautonomen Messgeräts mit einem Gehäuse, in dem zumindest
- ein Kernspinresonanz-Sensor,
- eine Steuervorrichtung zur Steuerung des Messgeräts,
- eine Auswertevorrichtung zur Auswertung eines von dem Kernspinresonanz-Sensor gelieferten Messsignals,
- eine Ausgabevorrichtung zur Ausgabe von ermittelten Informationen sowie
- eine Energieversorgungsvorrichtung des Messgeräts in Form einer Batterie, insbesondere einer wiederaufladbaren Batterie,
vorgesehen sind, zur Untersuchung eines Bestandteils eines menschlichen oder tierischen Körpers, insbesondere zur Untersuchung von Gewebe und/oder Körperflüssigkeiten, bevorzugt zur Untersuchung von Blut und/oder Urin, vorgeschlagen.

Unter einem "handgehaltenen Messgerät" soll hier insbesondere verstanden werden, dass das Messgerät ohne Zuhilfenahme einer Transportmaschine lediglich mit den Händen, insbesondere mit einer Hand, transportiert und insbesondere auch während eines Messvorgangs an einen und/oder entlang eines zu untersuchenden Bestandteils eines menschlichen oder tierischen Körpers geführt werden kann. Dazu beträgt die Masse des handgehaltenen Messgeräts insbesondere weniger als 20 kg, vorteilhaft weniger als 10 kg und besonders vorteilhaft weniger als 2 kg. In einer Ausführungsform weist das Messgerät einen Griff oder einen Griffbereich auf, mit dem das Messgerät über einen zu untersuchenden Bestandteil eines menschlichen oder tierischen Körpers geführt werden kann. Alternativ oder zusätzlich kann auch der zu untersuchende Bestandteil eines menschlichen oder tierischen Körpers zu dessen Untersuchung an das Messgerät und/oder entlang des Messgeräts geführt werden.

In einer Ausführungsform des handgehaltenen Messgeräts sind die Komponenten des Messgeräts, insbesondere der Kernspinresonanz-Sensor, die Steuervorrichtung, die Auswertevorrichtung sowie die Energieversorgungsvorrichtung des Messgeräts, zumindest teilweise in dem Gehäuse des Messgeräts untergebracht. Insbesondere sind die Komponenten in ihrem Gesamtvolumen zu mehr als 50 %, bevorzugt zu mehr als 75 % und besonders bevorzugt zu 100 % in dem Gehäuse des Messgeräts untergebracht. Bei einem derart gestalteten Messgerät kann eine vorteilhafte Verwendung durch leichtes, insbesondere einhändiges, Führen des Messgeräts an einen und/oder über einen zu untersuchenden Bestandteil eines menschlichen oder tierischen Körpers realisiert sein. Des Weiteren sind die Komponenten auf diese Weise vor Beschädigungen und Umwelteinflüssen, beispielsweise Feuchtigkeit und Staub, geschützt.

Das Messgerät ist als ein energieautonomes Messgerät realisiert. Unter "energieautonom" ist zu verstehen, dass das Messgerät zumindest vorübergehend, bevorzugt zumindest während der Dauer einer Untersuchung eines Bestandteils eines menschlichen oder tierischen Körpers, unabhängig von einem Stromnetz, d.h. insbesondere kabellos, betrieben werden kann. Dazu weist das Messgerät eine Energieversorgungsvorrichtung in Form eines stromnetzunabhängigen Energiespeichers, insbesondere in Form einer Batterie, bevorzugt in Form einer wiederaufladbaren Batterie, auf. Die Energieversorgungsvorrichtung ist dazu vorgesehen, das Messgerät zur Inbetriebnahme und während des Betriebs mit elektrischer Energie zu versorgen. Der stromnetzunabhängige Energiespeicher kann in einer alternativen Ausführungsform als eine Brennstoffzelle, ein Kondensator, ein hybrider Superkondensator oder als ein anderweitiger, dem Fachmann sinnvoll erscheinender Energiespeicher oder eine Kombination/Mehrung derer realisiert sein. Insbesondere eignen sich zur Energieversorgung des Messgeräts Akkumulatoren mit einer Zellchemie, die eine hohe Leistungs- und/oder Energiedichte bereitstellt. Eine hohe Leistungs- und/oder Energiedichte erlaubt eine verbesserte, d.h. langlebigere und an einen hohen Leistungsbedarf des Kernspinresonanz-Sensors angepasste, Energieversorgung des Messgeräts. Dazu gehören derzeit beispielsweise Akkumulatoren der Lithium- und Lithium-Ionen-Zellchemie, insbesondere Lithium-Eisenphosphat-, Lithium-Manganoxid-, Lithium-Nickel-Cobalt-Mangan-Oxid-, überlithiierte Lithium-Nickel-Cobalt-Mangan-Oxid-, Lithium-Schwefel-, Lithium-Polymer- und Lithium-Sauerstoff-Akkumulatoren. In einer Ausführungsform ist die Energieversorgungsvorrichtung mittels einer Formschluss- und/oder Kraftschlussverbindungsschnittstelle vom Messgerät lösbar realisiert. Unter "lösbar" soll in diesem Zusammenhang insbesondere zerstörungsfrei trennbar verstanden werden. Somit ist die Energieversorgungsvorrichtung bevorzugt abnehmbar und austauschbar an und/oder in dem Messgerät anordenbar. In Form eines derartigen Energiespeichers lässt sich die abnehmbare Energieversorgungsvorrichtung in und/oder außerhalb des Messgeräts wieder mit Energie aus einem Stromnetz versorgen und laden. In einer Ausführungsform der Energieversorgungsvorrichtung ist diese dazu vorgesehen, neben der Verwendung zur Energieversorgung des Messgeräts auch zur Energieversorgung anderer Geräte, insbesondere anderer Messgeräte und/oder anderer Handwerkzeugmaschinenvorrichtungen, nutzbar zu sein.

Unter "vorgesehen" soll insbesondere speziell "programmiert", "ausgelegt" und/oder "ausgestattet" verstanden werden. Darunter, dass ein Objekt zu einer bestimmten Funktion "vorgesehen" ist, soll insbesondere verstanden werden, dass das Objekt diese bestimmte Funktion in zumindest einem Anwendungs- und/oder Betriebszustand erfüllt und/oder ausführt oder dazu ausgelegt ist, die Funktion zu erfüllen.

Das mobile Messgerät weist eine Steuervorrichtung zu dessen Steuerung auf. Die Steuervorrichtung ist mit den anderen Komponenten des Messgeräts, insbesondere dem Kernspinresonanz-Sensor, der Auswertevorrichtung, der Ausgabevorrichtung, ferner beispielsweise einer Eingabevorrichtung, der Energieversorgungsvorrichtung sowie einer Datenkommunikationsschnittstelle, signaltechnisch verbunden. Die Steuervorrichtung ist dazu vorgesehen, während des Betriebs des Messgeräts mit diesen Komponenten zu kommunizieren. Unter der "Steuervorrichtung" soll insbesondere eine Vorrichtung mit zumindest einer Steuerelektronik verstanden werden, die Mittel zur Kommunikation mit den anderen Komponenten des Messgeräts, beispielsweise Mittel zur Steuerung und/oder Regelung des Kernspinresonanz-Sensors, Mittel zur Datenverarbeitung, Mittel zur Datenspeicherung und/oder weitere, dem Fachmann als sinnvoll erscheinende Mittel aufweist. In einer Ausführungsform ist unter der Steuerelektronik der Steuervorrichtung eine Prozessoreinheit in Verbindung mit einer Speichereinheit sowie mit einem in der Speichereinheit gespeicherten Betriebsprogramm zu verstehen, das während des Steuervorgangs ausgeführt wird. Insbesondere können die elektronischen Bauteile der Steuervorrichtung auf einer Platine (Leiterplatte) angeordnet sein, beispielsweise in Form eines Mikrokontrollers.

Zur Untersuchung eines Bestandteils eines menschlichen oder tierischen Körpers weist das Messgerät einen Kernspinresonanz-Sensor auf. Die Funktionsweise des Kernspinresonanz-Sensors basiert auf dem kernphysikalischen Effekt, bei dem Atomkerne in dem zu untersuchenden Bestandteil eines menschlichen oder tierischen Körpers in einem ersten Magnetfeld, bezeichnet mit B₀, elektromagnetische Wechselfelder absorbieren und emittieren. Dabei beruht die Kernspinresonanz auf der Präzession (Larmorpräzession) von Kernspins der Atomkerne in dem zu untersuchenden Bestandteil eines menschlichen oder tierischen Körpers um die Magnetfeldlinien des ersten, insbesondere konstanten und/oder statischen, Magnetfelds. Insbesondere werden die Kernspins der Atomkerne in dem zu untersuchenden Bestandteil eines menschlichen oder tierischen Körpers durch das erste Magnetfeld ausgerichtet. Wird Energie in Form eines zweiten elektromagnetischen Feldes, insbesondere eines Wechselfeldes, beispielsweise eines gepulsten Magnetfeldes, auf die Atomkerne eingestrahlt, die mit der Larmorpräzession deren Kernspins in Resonanz ist (Energiequanten), so können die Atomkerne die Orientierung ihrer Spins relativ zum ersten Magnetfeld durch Absorption dieser Energie ändern. Das zweite eingestrahlte Magnetfeld dient daher der Anregung der Kernspins, die unter Energieaufnahme ihre Kernspinzustände ändern. Äquivalent führt die Emission von Energiequanten in Folge einer Rückkehr der angeregten Kernspins in ein anderes, niedrigeres Energieniveau, zur Emission eines elektromagnetischen Wechselfeldes, welches sich mittels einer Vorrichtung zur Detektion einer Magnetfeldänderung, insbesondere mittels einer Antenne und/oder einer Spule, beobachten lässt. Unter den Atomkernen sind insbesondere Protonen (H) und andere kernspinresonanz-aktive Kerne wie beispielsweise 13C, 15N, 19F, 31P zu verstehen.

Der Kernspinresonanz-Sensor des Messgeräts erlaubt somit, Atomkerne in dem zu untersuchenden Bestandteil eines menschlichen oder tierischen Körpers mittels elektromagnetischer Wechselfelder anzuregen sowie ein Ausgangssignal aufgrund eines Kernspinresonanzeffektes zu generieren. Unter Anregung von Atomkernen soll insbesondere verstanden werden, dass die Energie der eingestrahlten elektromagnetischen Felder, insbesondere Wechselfelder, eine Änderung der Kernspins der Atomkerne bewirkt. Es sei angemerkt, dass im Folgenden davon ausgegangen wird, dass insbesondere veränderliche Magnetfelder mit elektrischen Feldern gekoppelt sind (vgl. Maxwell-Gleichungen), sodass keine Unterscheidung zwischen elektrischem Feld und Magnetfeld vorgenommen wird. Zur Anregung von Kernspinresonanz-Effekten kommt es auf die durch eine eingestrahlte elektromagnetische Strahlung übertragene Energie an. In einer Ausführungsform lässt sich diese Energie mittels gepulster elektromagnetischer Felder übertragen.

Bei geeigneter Wahl der Betriebsparameter des Kernspinresonanz-Sensors kann unter Verwendung des Messgeräts mittels des gemessenen Antwortsignals bei geeigneter Auswertung unmittelbar auf den Bestandteil eines menschlichen oder tierischen Körpers sowie auf dessen Eigenschaften, zumindest in einem untersuchten Volumen des Bestandteils eines menschlichen oder tierischen Körpers, geschlossen werden. Die Eigenschaften können dabei beispielsweise die Zusammensetzung des Bestandteils, das Vorhandensein von Stoffkomponenten sowie deren Konzentration oder dergleichen betreffen.

Unter der "Auswertevorrichtung" zur Auswertung zumindest eines von dem Kernspinresonanz-Sensor gelieferten Messsignals soll zumindest eine Vorrichtung verstanden werden, die einen Informationseingang zur Annahme der Messsignale des Kernspinresonanz-Sensors, eine Informationsverarbeitungseinheit zur Bearbeitung, insbesondere Auswertung der angenommenen Messsignale, sowie eine Informationsausgabe zur Weitergabe der bearbeiteten und/oder ausgewerteten Messsignale aufweist. In einer Ausführungsform weist die Auswerteeinheit Komponenten auf, die zumindest einen Prozessor, einen Speicher und ein Betriebsprogramm mit Auswerte- und Berechnungsroutinen umfassen. Insbesondere können die elektronischen Bauteile der Auswertevorrichtung auf einer Platine (Leiterplatte) angeordnet sein, insbesondere auf einer gemeinsamen Platine mit der Steuervorrichtung. In einer Ausführungsform kann die Auswertevorrichtung in Form eines Mikrokontrollers realisiert sein. Des Weiteren können die Steuervorrichtung und die Auswertevorrichtung als ein einzelnes Bauteil ausgeführt sein. Die Auswertevorrichtung ist vorgesehen, die von dem Kernspinresonanz-Sensor erhaltenen Messsignale auszuwerten und daraus zumindest Informationen betreffend den untersuchten Bestandteil eines menschlichen oder tierischen Körpers abzuleiten.

Unter der "Ausgabevorrichtung" des Messgeräts soll zumindest ein Mittel verstanden werden, das dazu vorgesehen ist, zumindest eine wechselnde Information akustisch, optisch und/oder taktil an einen Bediener auszugeben. Die Ausgabevorrichtung dient der Ausgabe zumindest derjenigen Informationen an den Bediener des Messgeräts, die unter Verwendung des Messgeräts über den untersuchten Bestandteil eines menschlichen oder tierischen Körpers erhalten werden. Die Ausgabe kann dabei beispielsweise mittels eines Displays, eines Touch-Displays, eines Tonsignals, eines Vibrationsgebers und/oder einer LED-Anzeige realisiert werden. In einer Ausführungsform der Ausgabevorrichtung kann die Information grafisch oder alphanumerisch als Messergebnis der Untersuchung ausgegeben werden. Die Ausgabevorrichtung ist im Gehäuse des handgehaltenen Messgeräts untergebracht. Ferner können auszugebende Informationen, beispielsweise Informationen über einen untersuchten Bestandteil, auch an die Steuervorrichtung und/oder, insbesondere zur Erhöhung des Nutzerkomforts, an ein Daten verarbeitendes System ausgegeben werden. Letzteres umfasst zumindest eine Ausgabe einer Information an ein externes Geräte wie ein Smartphone, ein Tablet-PC, ein PC sowie an ein anderes, einem Fachmann als sinnvoll erscheinendes externes Datengerät, das über eine Datenkommunikationsschnittstelle mit der Auswertevorrichtung des Messgeräts verbunden ist. Somit ist die Ausgabevorrichtung direkt im Gehäuse des Messgeräts untergebracht und kann zusätzlich auch über externe Ausgabevorrichtungen ergänzt werden. Letztere Realisierungsmöglichkeit umfasst explizit die Steuerung, Auswertung und Ausgabe der ermittelten Informationen über drahtgebundene und/oder drahtlose externe Systeme wie beispielsweise Fernbedienungen, Computersteuerungen, Tablet-PCs und/oder andere mobile Geräte wie Mobiltelefone, Smartphones etc.

Unter "Bestandteil eines menschlichen oder tierischen Körpers" soll jeglicher materielle Teil des Körpers im biologischen Sinne verstanden werden (im Unterschied zu rein geometrischen oder physikalischen Definitionen), d.h. jeder materielle Teil des Körpers als Organismus, unabhängig davon, ob er lebt oder nicht. Somit stellt der Bestandteil eines menschlichen oder tierischen Körpers ein stoffliches Gebilde dar, das prinzipiell in unterschiedlichem Zusammenhang mit dem menschlichen oder tierischen Körper stehen kann. Beispielsweise kann der Bestandteil in anatomischem Zusammenhang mit der Struktur und/oder dem Aufbau des menschlichen oder tierischen Körpers stehen oder in physiologischem Zusammenhang mit den Körperfunktionen und den dabei innerhalb eines Körpers ablaufenden Stoffwechselprozessen. Ferner kann der Bestandteil auch im Zusammenhang mit biochemischen Prozessen auf mikrobiologischer Ebene stehen. Der Begriff "Bestandteil eines menschlichen oder tierischen Körpers" umfasst dabei sowohl flüssige als auch feste Bestandteile. Beispiele eines derartig definierten Bestandteils eines menschlichen oder tierischen Körpers sind Arme, Beine, Kopf (Körperteile), Haut, Gewebeproben, Nagel, Knochen, Gehirn, Körperflüssigkeiten wie Speichel, Blut und/oder Urin, Fäzes, Eiter, Furunkel, Abszesse oder dergleichen. Es sei darauf hingewiesen, dass sich der zu untersuchende Bestandteil eines menschlichen oder tierischen Körpers sowohl im oder am lebendigen Körper (Organismus), im oder am toten Körper als auch außerhalb eines menschlichen oder tierischen Körpers, insbesondere in künstlicher Umgebung (z. B. im Reagenzglas), befinden kann.

Unter "Verwendung zur Untersuchung eines Bestandteils eines menschlichen oder tierischen Körpers" soll insbesondere verstanden werden, unter Verwendung des Messgeräts aus den von dem Kernspinresonanz-Sensor erhaltenen Messsignalen Informationen zu bestimmen und somit Aussagen abzuleiten, die vielfältige Eigenschaften und insbesondere den Zustand des Bestandteils eines menschlichen oder tierischen Körpers betreffen. Diese Aussagen bzw. Informationen können insbesondere die Struktur, Zusammensetzung, Steuervorgänge sowie Stoffwechselprozesse des menschlichen oder tierischen Körpers betreffen, aus denen sich bevorzugt Aussagen zu Zuständen, Erkrankungen, Funktionsstörungen, möglichen Heilungsprozessen oder -ansätzen oder dergleichen des Körpers ableiten lassen. Insbesondere lassen sich bei geeigneter Auswertung der Messsignale des Kernspinresonanz-Sensors Informationen hinsichtlich in dem Bestandteil vorhandener Substanzen und deren Konzentration ermitteln. Derart kann unter Verwendung des Messgeräts beispielsweise Blut und/oder Urin auf Entzündungsindikatoren, Krebsindikatoren, Nierenwerte, Mineralwerte, Drogen-Indikatoren, Blutzuckergehalt, Sauerstoffgehalt, Cholesteringehalt, Konzentrationen von Lipoprotein niedriger/hoher Dichte (LDL, HDL) oder andere Analyten untersucht werden. Ferner ist eine Untersuchung einer Venenfunktion (im Zusammenhang mit Arteriosklerose, Thrombose), eine Untersuchung von Verbänden auf Nachblutung ohne Öffnung des Verbands, eine Bestimmung einer Blutgruppe, das Aufspüren innerer Blutungen in Organen, eine Untersuchung von Wasseransammlungen (Ödeme) im Körper denkbar.

In einer Ausführungsform, insbesondere bei der Bestimmung von Analytkonzentrationen, kann eine Untersuchung quantitativ erfolgen. Es sei darauf hingewiesen, dass der zu untersuchende Bestandteil eines menschlichen oder tierischen Körpers sowohl im oder am lebenden Körper, in oder am toten Körper als auch außerhalb eines menschlichen oder tierischen Körpers, insbesondere in künstlicher Umgebung (z. B. im Reagenzglas), untersucht werden kann.

Aus derartigen ausgewerteten Informationen kann ein Bediener des Messgeräts einen Zustand, eine Behandlungsfälligkeit, eine Behandlungsfähigkeit oder dergleichen des menschlichen oder tierischen Körpers auf einfache Weise untersuchen und prüfen. Insbesondere sind Indikatoren für eine medizinische Diagnose bestimmbar.

Zur Durchführung der Messung wird das mobile Messgerät, insbesondere der Kernspinresonanz-Sensor, nahe an den zu untersuchenden Bestandteil eines menschlichen oder tierischen Körpers herangebracht oder umgekehrt. Die Verwendung des Messgeräts erlaubt dabei die Untersuchung eines Bestandteils eines menschlichen oder tierischen Körpers ohne Beeinträchtigung des Bestandteils, d.h. insbesondere ohne Zerstörung, Kontamination oder dergleichen. Bei dem Kernspinresonanz-Messverfahren handelt es sich um ein nicht zerstörendes, insbesondere kontaktloses Messverfahren, sodass ein Bestandteil eines menschlichen oder tierischen Körpers ohne jeglichen Kontakt des Messgeräts zu dem Bestandteil untersucht werden kann. Insbesondere kann eine Untersuchung unter Verwendung des Messgeräts nicht-invasiv erfolgen. Das Positionieren des Messgerätes, insbesondere des darin enthaltenen Kernspinresonanz-Sensors, in unmittelbare Nähe eines zu untersuchenden Bestandteils eines menschlichen oder tierischen Körpers (oder umgekehrt) ermöglicht die Untersuchung des Bestandteils eines menschlichen oder tierischen Körpers bis zu einer Materialtiefe von einigen Zentimeter in den Bestandteil hinein. Somit kann ebenfalls eine subkutane Untersuchung eines Bestandteils eines menschlichen oder tierischen Körpers ohne Zerstörung (Öffnen) der Haut erfolgen.

Das handgehaltene, energieautonome Messgerät stellt ein spezialisiertes Messgerät dar, das im Vergleich zu wissenschaftlichen Kernspinresonanz-Messgeräten eine stark eingeschränkte, auf die Untersuchung eines Bestandteils eines menschlichen oder tierischen Körpers optimierte Funktionalität aufweist. Insbesondere die Auswertevorrichtung mit ihren Auswerteroutinen ist auf die Untersuchung von Bestandteilen eines menschlichen oder tierischen Körpers, die Bewertung der erhaltenen Informationen sowie deren aufbereiteter Darstellung und Ausgabe mittels der Ausgabevorrichtung zugeschnitten. Bei Verwendung des Messgeräts zur Untersuchung eines Bestandteils eines menschlichen oder tierischen Körpers werden die Messergebnisse, d.h. die den untersuchten Bestandteil eines menschlichen oder tierischen Körpers betreffenden Informationen, für den Bediener des Messgeräts geräteintern und der Messung unmittelbar folgend aufbereitet, sodass eine schnelle, eindeutige und vor allem von weiteren Geräten wie Computern oder gar von Laboratorien unabhängige Beurteilung des untersuchten Bestandteils eines menschlichen oder tierischen Körpers vor Ort möglich ist. Vorteilhaft ist eine einfache und intuitive Bedienung des Messgeräts erreichbar, die keine besondere Vorerfahrung des Bedieners voraussetzt.

Darüber hinaus sind die Baugröße, die Energieversorgung sowie die Konstruktion des Messgeräts hinsichtlich Anordnung des Kernspinresonanz-Sensors an die mobile, handgehaltene und energieautonome Verwendung des Messgeräts zur Untersuchung von Bestandteilen eines menschlichen oder tierischen Körpers angepasst. In einer Ausführungsform des Messgeräts weist dieses eine plane Auflagefläche auf, an die ein Bestandteil eines menschlichen oder tierischen Körpers angelegt werden kann (oder umgekehrt). In einer alternativen oder zusätzlichen Ausführungsform des Messgeräts weist dieses in dem Gehäuse eine Aufnahme für eine zu untersuchende Probe, insbesondere für ein Reagenzglas oder dergleichen, und/oder eine Aufnahme für einen zu untersuchenden Bestandteil eines menschlichen oder tierischen Körpers, beispielsweise Extremitäten wie ein Finger, auf. Diese Aufnahme kann in einer Ausführungsform rund und/oder zylindrisch ausgestaltet sein. In einer alternativen Ausführungsform kann diese Aufnahme auch eine offene, C-förmige Form aufweisen.

Durch Verwendung des handgehaltenen, energieautonomen und speziell auf den Anwendungsfall der Untersuchung von Bestandteilen eines menschlichen oder tierischen Körpers zugeschnittenen Messgeräts zur Untersuchung des Bestandteils ist es möglich, auf schnelle und zerstörungsfreie Weise und damit wirtschaftlich besonders kostengünstig eine präzise und umfassende Untersuchung des Bestandteils eines menschlichen oder tierischen Körpers zu realisieren. Vorteilhaft ermöglicht die erfindungsgemäße Verwendung des handgehaltenen, energieautonomen Messgeräts die schnelle und präzise Untersuchung von Bestandteilen eines menschlichen oder tierischen Körpers mobil an beliebigen Orten und insbesondere unabhängig von einem Labor.

In einer Ausgestaltung wird das Messgerät zur Untersuchung eines Bestandteils eines menschlichen oder tierischen Körpers verwendet, wobei mittels der Auswertevorrichtung des Messgeräts das von dem Kernspinresonanz-Sensor gelieferte Messsignal, insbesondere ein Spektrum und/oder eine Relaxationszeit des Messsignals resultierend aus der Anregung von Kernspins in dem zu untersuchenden Bestandteil eines menschlichen oder tierischen Körpers durch den Kernspinresonanz-Sensor, ausgewertet wird.

Erfindungsgemäß wird das mobile Messgerät dazu verwendet, einen Bestandteil eines menschlichen oder tierischen Körpers insbesondere hinsichtlich verschiedener Merkmale wie Zusammensetzung, Konzentration von enthaltenen Substanzen und/oder medizinisch interessanter Zustände oder dergleichen umfassend zu charakterisieren und insbesondere Indikatoren für eine medizinische Diagnose zu bestimmen. Dabei lassen sich bei Verwendung des Messgeräts mittels des Kernspinresonanz-Sensors Spektren und/oder Relaxationszeiten messen, die eine von dem untersuchten Bestandteil abhängige Signatur bzw. Charakteristik aufweisen, genauer gesagt, eine von dem atomaren Aufbau des Bestandteils abhängige Signatur bzw. Charakteristik aufweisen. Die Auswertevorrichtung ist speziell zur zügigen Auswertung der von dem Kernspinresonanz-Sensor gelieferten Messsignale ausgebildet. Zügig bedeutet insbesondere innerhalb von 10 Minuten, bevorzugt innerhalb von 60 Sekunden, besonders bevorzugt innerhalb von 5 Sekunden.

Bei geeigneter Wahl der Betriebsparameter des Kernspinresonanz-Sensors kann mittels eines Spektrums und/oder Relaxationszeiten des Antwortsignals unmittelbar auf Eigenschaften des zu untersuchenden Bestandteils eines menschlichen oder tierischen Körpers geschlossen werden. In einem Chemometrie-Ansatz kann dabei beispielsweise durch Auswertung mittels einer Hauptkomponentenanalyse (PCA) eine Untersuchung des Bestandteils eines menschlichen oder tierischen Körpers erfolgen, ohne konkrete Ursache-Wirkungszusammenhänge zu kennen. Dabei werden das Spektrum oder mehrere Spektren, chemische Verschiebungen, Kopplungskonstanten, Korrelationen und/oder Relaxationszeiten oder dergleichen als Eingangsdaten der Auswertung verwendet. In Folge der Auswertung werden Punktwolken bzw. grafisch darstellbare Bereiche erhalten, die sich durch Vergleich mit Referenzdaten auf einfache und zügige Weise weiter auswerten und insbesondere interpretieren lassen. Beispielsweise kann in einer Verwendungsform des Messgerät mittels der Auswertevorrichtung aus den mit dem Kernspinresonanz-Sensor erhaltenen Messsignalen zumindest eine Information aus einer Liste von Informationen ausgewertet, insbesondere tiefenaufgelöst ausgewertet werden, wobei die Liste zumindest
- einen relativen und/oder absoluten Kohlenwasserstoffgehalt und/oder
- Bindungszustände chemischer Verbindungen und/oder
- einen Konzentrationsgradienten eines Materials und/oder einer Verbindung und/oder eines Elements in den Bestandteil eines menschlichen oder tierischen Körpers hinein und/oder
- zeitlich-dynamische Prozesse chemischer Verbindungen und/oder
- einen relativen und/oder absoluten Feuchtegehalt und/oder
- weitere biochemisch relevante Parameter
des Bestandteils eines menschlichen oder tierischen Körpers umfasst.

Aussagen zu den Bindungszuständen in dem Bestandteil eines menschlichen oder tierischen Körpers erlauben festzustellen, um welches Material oder welche Materialien bzw. Substanzen es sich in dem Bestandteil eines menschlichen oder tierischen Körpers handelt. Beispielsweise lassen sich auf diese Weise unterschiedliche Substanzen, Einschlüsse oder dergleichen detektieren und unterscheiden. Ebenfalls kann eine Bestimmung einer Materialkonzentration in dem untersuchten Bestandteil realisiert werden, sofern eine Kalibrierung des Kernspinresonanz-Sensors vor der Messung erfolgt. Derart lassen sich Wassergehalte, Fettgehalte (HDL, LDL), Zuckergehalte, Cholesteringehalte, Drogen-/Alkoholgehalte, Mineralgehalte, Allergengehalte, Toxingehalte oder beliebige andere, einem Fachmann sinnvoll erscheinende Gehalte oder Konzentrationen in einem Bestandteil eines menschlichen oder tierischen Körpers unter Verwendung des Messgeräts ermitteln. In einer Ausführungsform kann eine Material- bzw. Substanzkonzentration mit einem zulässigen Grenzwert (Schwellenwert) verglichen werden und bei Überschreiten des Grenzwerts eine Warnung durch das Messgerät ausgegeben werden.

Mit der Aufnahme und Auswertung zeitlich-dynamischer Prozesse chemischer Verbindungen können im Bestandteil des menschlichen oder tierischen Körpers ablaufende Prozesse wie beispielsweise Verdauung, innere Blutung oder dergleichen untersucht werden. Darüber hinaus kann das Messgerät ebenfalls eingesetzt werden, einen Bestandteil eines menschlichen oder tierischen Körpers hinsichtlich Feuchtigkeit umfassend zu charakterisieren. Aussagen über den relativen und/oder absoluten Feuchtegehalt sowie über einen Feuchtegradienten in den Bestandteil eines menschlichen oder tierischen Körpers hinein ermöglichen eine zuverlässige Bewertung des Bestandteils eines menschlichen oder tierischen Körpers insbesondere hinsichtlich Vorhandensein von Wasseransammlungen, Austrocknung oder dergleichen.

Weitere biochemisch relevante Parameter, die unter Verwendung des Messgeräts mit der Auswertevorrichtung ausgewertet werden können, umfassen beispielsweise eine Dichte und/oder Porosität von Knochen in dem untersuchten Bestandteil.

Je nach gewünschter Information misst der Kernspinresonanz-Sensor ein Spektrum und/oder Relaxationskurven und/oder Relaxationszeiten, wobei die Auswertevorrichtung die gezielte Auswertung dieser Messsignale hinsichtlich der gewünschten Information durchführt.

In einer Ausgestaltung wird das Messgerät zur Untersuchung eines Bestandteils eines menschlichen oder tierischen Körpers verwendet, wobei mittels der Auswertevorrichtung des Messgeräts eine ermittelte Information, insbesondere ein Spektrum und/oder eine Relaxationszeit, mit Referenzdaten einer Referenzdatenbank verglichen wird.

Auf diese Weise kann eine besonders einfache und umfassende Auswertung und/oder Bewertung und/oder Interpretation eines gemessenen Messsignals des Kernspinresonanz-Sensors erfolgen. Beispielsweise kann ein aufgenommenes Spektrum und/oder aufgenommene Relaxationskurven, insbesondere Relaxationszeiten, mit Referenzspektren, Referenzrelaxationskurven bzw. Referenzrelaxationszeiten verglichen werden. Der Vergleich der Messsignale mit im Detail bestimmten und bekannten Referenzdaten dient dabei der schnellen und einfachen Zuordnung des Messsignals, um eine bestimmte Information aus dem gemessenen Messsignal abzuleiten. Durch den Vergleich mit bekannten Referenzdaten kann ferner - neben einer sehr hohen Auswertegeschwindigkeit - ein besonders genaues Ergebnis, d.h. eine besonderes genaue Information über den untersuchten Bestandteil eines menschlichen oder tierischen Körpers erhalten werden. Beispielsweise kann in einem Vergleich mit einem Referenzspektrum eine Verschiebung eines gemessenen Spektrums sehr leicht und schnell erkannt werden, sodass aus dieser Verschiebung eine Information betreffend den untersuchten Bestandteil ableitbar ist.

Vorteilhaft können durch den Vergleich der gemessenen Messsignale mit Referenzdaten auf besonders einfache Weise Abweichungen der Messsignale von den Referenzdaten identifiziert werden. Übersteigen diese Abweichungen eine definierte Toleranzschwelle, können sie als Indiz für Unregelmäßigkeit und/oder Unstimmigkeiten betreffend den untersuchten Bestandteil eines menschlichen oder tierischen Körpers interpretiert werden. Insbesondere lassen sich aus Unstimmigkeiten und/oder Unregelmäßigkeiten Rückschlüsse auf mögliche Erkrankungen oder Anomalien, insbesondere Stoffwechselerkrankungen, des menschlichen oder tierischen Körpers und/oder über eine Ernährung oder dergleichen ziehen.

Die Referenzdaten, insbesondere Referenzkurven und/oder Referenzwerte und/oder Referenzspektren und/oder Referenzbilder, können dabei geräteintern in einer Datenbank auf einer Speichereinheit, insbesondere einer Speichereinheit der Steuer- und/oder der Auswertevorrichtung, gespeichert sein. In einer alternativen oder zusätzlichen Ausführungsform können die Referenzdaten auch in einer geräteexternen, vorteilhaft stets aktuellen, Referenzdatenbank gespeichert sein, beispielsweise in einer Referenzdatenbank auf einem Computer, einem Server oder auf einem anderen, einem Fachmann sinnvoll erscheinenden Datenspeicher und/oder Datenverarbeitungsgerät. Insbesondere kann der Vergleich der gemessenen Messsignale mit den Referenzdaten über einen Internetzugang des Messgeräts erfolgen. Alternativ oder zusätzlich können geräteintern gespeicherte Referenzdaten ebenfalls über einen Internetzugang des Messgeräts aktualisiert werden, beispielsweise durch einen Abgleich mit einer geräteexternen Referenzdatenbank.

In einer Ausgestaltung wird das Messgerät zur Untersuchung eines Bestandteils eines menschlichen oder tierischen Körpers verwendet, wobei mittels der Ausgabevorrichtung des Messgeräts, insbesondere mittels eines Displays, eine ermittelte Information, insbesondere ein Spektrum und/oder eine Relaxationszeit, und/oder eine Abweichung einer ermittelten Information von Referenzdaten einer Referenzdatenbank, ausgegeben werden/wird, insbesondere dargestellt werden/wird.

Unter Verwendung der mittels der Ausgabevorrichtung dargestellten Information ist es dem Bediener des Messgeräts möglich, nach Durchführung der Untersuchung des Bestandteils eines menschlichen oder tierischen Körpers zu einem intuitiv verständlichen Ergebnis zu gelangen.

In einer bevorzugten Ausführungsform werden die den untersuchten Bestandteil betreffenden Informationen in einer intuitiv verständlichen, insbesondere aufbereiteten Weise dem Bediener des Messgeräts ausgegeben. Intuitiv ist die Ausgabe insbesondere dann, wenn der Bediener ohne Vorkenntnisse eine Untersuchung eines Bestandteils eines menschlichen oder tierischen Körpers durchführen kann und anschließend eine Aussage, bevorzugt eine Bewertung, aus den angezeigten Informationen ableiten kann. In einer Ausführungsform kann eine Aufbereitung der ermittelten Information beispielsweise in Form einer Farbzuordnung erfolgen. Dabei kann beispielsweise Rot eine ermittelte bedenkliche Abweichung einer untersuchten Zielgröße - beispielsweise einer Konzentration - von einer Vorgabe - beispielsweise definiert durch die Referenzdaten in der Referenzdatenbank - signalisieren. Gelb hingegen signalisiert eine Abweichung innerhalb einer zulässigen Toleranz und Grün eine ermittelte Abweichung in zulässigem und/oder unbedenklichem Maße. Alternativ oder zusätzlich kann eine Ausgabe der ermittelten Information in Form einer Kurznachricht erfolgen, die auf dem Display dargestellt wird. Diese Kurznachricht kann beispielsweise eine Spezifikation der zur Bewertung des untersuchten Bestandteils eines menschlichen oder tierischen Körpers herangezogenen Referenzdaten beinhalten, ferner eine Bewertung der Abweichung, sofern ermittelt, sowie daraus abgeleitete Empfehlungen wie "Insulin spritzen" oder "Auf magnesiumreiche Ernährung achten" oder dergleichen.

In einer Ausgestaltung wird das Messgerät zur Untersuchung eines Bestandteils eines menschlichen oder tierischen Körpers verwendet, wobei mittels des Messgeräts der Bestandteil eines menschlichen oder tierischen Körpers ortsaufgelöst, insbesondere positionsaufgelöst und/oder tiefenaufgelöst, untersucht wird.

Eine lateral ortsaufgelöste Untersuchung kann beispielsweise unter Verwendung einer Positionsbestimmungsvorrichtung des Messgeräts zur Erfassung zumindest einer momentanen Position des Messgeräts, insbesondere bezogen auf den Bestandteil und/oder bezogen auf den Körper, erfolgen. Unter der Position ist ebenfalls eine Ausrichtung des Messgeräts, insbesondere bezogen auf den Bestandteil und/oder bezogen auf den Körper, zu verstehen. Die Positionsbestimmungsvorrichtung des Messgeräts kann dazu beispielsweise insbesondere einen oder mehrere Sensoren aus einer Gruppe von Sensoren aufweisen, die zumindest neigungs-, winkel-, abstands-, translations-, beschleunigungs- sowie drehratensensitive Sensoren umfasst. Beispielsweise kann die Positionsbestimmungsvorrichtung unter Verwendung von am Gehäuse des Messgeräts angeordneten Rädern realisiert sein, die beim Verfahren des Messgeräts bezogen auf den Bestandteil eines menschlichen oder tierischen Körpers die Positionsänderung aufnehmen. In einer alternativen oder zusätzlichen Ausführungsform kann eine ortsaufgelöste Untersuchung auch mittels eines elektrisch durch Ansteuerung schwenkbaren und/oder mittels eines mechanisch schwenkbaren Kernspinresonanz-Sensors erfolgen. Dabei stellt ein mechanisch schwenkbarer Kernspinresonanz-Sensor eine besonders einfache Realisierung eines richtungs- und somit ortsauflösenden Kernspinresonanz-Sensors dar. In einer Ausführungsform kann der mechanisch schwenkbare Kernspinresonanz-Sensor auch unter Verwendung einer elektrischen Schaltung in Verbindung mit Motoren oder vergleichbaren Aktuatoren automatisiert geschwenkt werden. Ferner kann der Kernspinresonanz-Sensor auch in Folge seiner elektrischen Ansteuerung ortsaufgelöst messen, indem beispielsweise eine definierte Verzerrung des ersten Magnetfelds mittels Gradientenspulen (Shim-Spulen) erfolgt.

Eine tiefenaufgelöste Untersuchung des Bestandteils eines menschlichen oder tierischen Körpers kann beispielsweise erfolgen, indem der Kernspinresonanz-Sensor in Richtung der Tiefe mechanisch linear verschoben oder verfahren wird. Auf diese Weise kann erreicht werden, dass der für den Kernspinresonanz-Sensor charakteristische sensitive Bereich im Innern des zu untersuchenden Bestandteils eines menschlichen oder tierischen Körpers verschoben wird, sodass auf einfache und besonders wirtschaftliche Weise eine tiefenaufgelöste Messung realisiert werden kann. Alternativ kann über eine Änderung der Frequenz, mit der das elektromagnetische Wechselfeld in den zu untersuchenden Bestandteil eines menschlichen oder tierischen Körpers eingestrahlt wird, der sensitive Bereich in dem Bestandteil, aus dem mittels des Kernspinresonanz-Sensors Messsignale empfangen werden, in der Tiefe - d.h. in Richtung in den zu untersuchenden Bestandteil eines menschlichen oder tierischen Körpers hinein - verschoben werden.

Bei Verwendung des Messgeräts zur ortsaufgelösten Untersuchung eines Bestandteils eines menschlichen oder tierischen Körpers werden mittels der Auswertevorrichtung Messsignale des Kernspinresonanz-Sensors in Abhängigkeit der Position des Messgeräts, insbesondere bezogen auf den Bestandteil, und/oder in Abhängigkeit der Tiefe in den Bestandteil eines menschlichen oder tierischen Körpers hinein ausgewertet. Somit kann erreicht werden, dass ausgewertete Informationen mit einer Position des Messgeräts auf dem Bestandteil eines menschlichen oder tierischen Körpers korreliert werden können. Ferner lassen sich durch sukzessives Umpositionieren des Messgeräts bezogen auf den Bestandteil eines menschlichen oder tierischen Körpers mehrdimensionale Karten, in denen ausgewertete Informationen zu Positionen des Messgeräts, insbesondere bezogen auf den Bestandteil, erfasst sind, erstellen. Analog kann eine tiefenaufgelöste Information ermittelt werden, aus der beispielsweise Verläufe in dem Bestandteil, d.h. in den Bestandteil hinein, entnehmbar sind. Derart lassen sich in einer Ausführungsform beispielsweise Feuchtigkeitsverläufe oder Konzentrationsverläufe in den Bestandteil eines menschlichen oder tierischen Körpers hinein ermitteln.

Eine unter Verwendung des Messgeräts ortsaufgelöst, insbesondere positionsaufgelöst und/oder tiefenaufgelöst, durchgeführte Untersuchung eines Bestandteils eines menschlichen oder tierischen Körpers erlaubt, auf besonders effektive und effiziente Weise an umfassende Informationen über den Bestandteil eines menschlichen oder tierischen Körpers zu gelangen. Beispielsweise kann mittels einer Relativ- oder Vergleichsmessung, bei der das Messgerät über den Bestandteil eines menschlichen oder tierischen Körpers bewegt wird, eine Untersuchung auf Grundlage positionsabhängiger Schwankungen der von dem Kernspinresonanz-Sensor gelieferten Messsignale erfolgen. In dem Bestandteil eines menschlichen oder tierischen Körpers verborgene Unregelmäßigkeiten und/oder Unstimmigkeiten führen beim Bewegen des Messgeräts über den Bestandteil zu eindeutigen, positionsabhängigen Änderungen der Messsignale. Auf Grundlage einer derartigen Vergleichsmessung lassen sich auf einfache und schnelle Weise Informationen über eine Substanzverteilung, beispielsweise eines Toxins, in dem untersuchten Bestandteil eines menschlichen oder tierischen Körpers ermitteln.

Ferner kann in Folge einer tiefenaufgelösten Untersuchung eines Bestandteils eines menschlichen oder tierischen Körpers eine Verteilung einer Substanz besonders gut ermittelt werden. Dabei kann in Folge einer tiefenaufgelösten Untersuchung eines Bestandteils eines menschlichen oder tierischen Körpers ein Gradient einer ausgewerteten Eigenschaft in den Bestandteil eines menschlichen oder tierischen Körpers hinein ermittelt werden. In einer Ausführungsform kann beispielsweise ein Konzentrations-Gradient in den Bestandteil eines menschlichen oder tierischen Körpers hinein zerstörungsfrei bestimmt werden.

In einer Ausgestaltung wird das Messgerät zur Untersuchung eines Bestandteils eines menschlichen oder tierischen Körpers verwendet, wobei mittels einer Eingabevorrichtung Angaben zu dem zu untersuchenden Bestandteil eines menschlichen oder tierischen Körpers durch Bedienereingaben spezifiziert und dem Messgerät zur Verfügung gestellt werden.

Unter einer Eingabevorrichtung soll insbesondere ein Mittel verstanden werden, das dazu vorgesehen ist, zumindest eine Information von einem Bediener des Messgeräts über eine akustische, optische, gestengestützte und/oder taktile Eingabe anzunehmen und an die Steuervorrichtung des Messgeräts weiterzuleiten. Beispielsweise kann die Eingabevorrichtung aus einem Betätigungselement, einer Tastatur, einem Display, insbesondere einem Touch-Display, einem Spracheingabemodul, einer Gestenerkennungseinheit und/oder einem Zeigegerät (beispielsweise einer Maus) bestehen. Alternativ oder zusätzlich kann die Eingabevorrichtung auch außerhalb des Messgeräts realisiert sein, beispielsweise in Form eines externen Datenverarbeitungsgeräts wie einem Smartphone, einem Tablet-PC, einem PC, oder dergleichen, das über eine Datenkommunikationsschnittstelle mit der Steuervorrichtung des Messgeräts verbunden ist.

Durch die Eingabe von Angaben zu einem Bestandteil eines menschlichen oder tierischen Körpers lassen sich eine Informationsverarbeitung, insbesondere die Auswertung des Messsignals, der Vergleich eines Messsignals und/oder einer ermittelten Information mit Referenzdaten oder dergleichen, vorteilhaft an den zu untersuchenden Bestandteil eines menschlichen oder tierischen Körpers anpassen. Beispielsweise kann in Abhängigkeit einer Bedienereingabe eine Referenzdatenbank gewählt werden. Ferner können insbesondere im Zusammenhang mit der Spezifikation ein Betriebsprogramm der Steuervorrichtung, Regelroutinen, Steuerroutinen, Auswerteroutinen und/oder Berechnungsroutinen angepasst werden.

"Angaben zu einem Bestandteil eines menschlichen oder tierischen Körpers" können beispielsweise den Bestandteil selbst charakterisieren ("Blut", "Urin", "Finger", "Arm"), den Körper charakterisieren ("männlich", "weiblich", Alter, Gewicht) und/oder weitere zur Auswertung und/oder Interpretation der Messsignale benötigte oder sinnvolle Werte betreffen. Alternativ oder zusätzlich können weitere, insbesondere die physikalischen und/oder chemischen Eigenschaften des Bestandteils eines menschlichen oder tierischen Körpers betreffende Angaben sinnvoll und/oder notwendig sein, wie beispielsweise Angaben zu "fest oder flüssig", "Dichte", oder dergleichen.

In einer Ausgestaltung wird das Messgerät zur Untersuchung eines Bestandteils eines menschlichen oder tierischen Körpers verwendet, wobei vor Untersuchung des Bestandteils eines menschlichen oder tierischen Körpers unter Verwendung einer geräteintern vorgesehenen Standardprobe, insbesondere unter Verwendung einer geräteintern vorgesehenen Tetramethylsilan-Probe, eine Kalibrierung des Messgeräts durchführt wird.

Zur genaueren Untersuchung eines Bestandteils eines menschlichen oder tierischen Körpers kann somit vor Durchführung der Untersuchung eine Kalibrierung des Messgeräts, insbesondere eine Kalibrierung des Kernspinresonanz-Sensors, erfolgen. Die Kalibrierung erfolgt in einer Ausführungsform unter Verwendung einer reinen Materialprobe, bevorzugt unter Verwendung einer, insbesondere geräteintern vorgesehenen, Tetramethylsilan-Probe (TMS), die als Standard verwendet wird. Alle im Anschluss an die Kalibrierung folgenden Messungen, insbesondere Messungen an einem zu untersuchenden Bestandteil eines menschlichen oder tierischen Körpers, werden in Bezug zu dieser Kalibriermessung ausgewertet.

Des Weiteren wird ein erfindungsgemäßes handgehaltenes, energieautonomes Messgerät zur Untersuchung eines Bestandteils eines menschlichen oder tierischen Körpers vorgeschlagen, das ein Gehäuse umfasst, in dem zumindest
- ein Kernspinresonanz-Sensor,
- eine Steuervorrichtung zur Steuerung des Messgeräts,
- eine Auswertevorrichtung zur Auswertung eines von dem Kernspinresonanz-Sensor gelieferten Messsignals,
- eine Ausgabevorrichtung zur Ausgabe von ermittelten Informationen sowie
- eine Energieversorgungsvorrichtung in Form einer Batterie, insbesondere einer wiederaufladbaren Batterie
vorgesehen sind, wobei das Messgerät, insbesondere der Kernspinresonanz-Sensor und/oder die Auswertevorrichtung, zur Untersuchung eines Bestandteils eines menschlichen oder tierischen Körpers, insbesondere zur Untersuchung von Gewebe und/oder Körperflüssigkeiten, bevorzugt zur Untersuchung von Blut und/oder Urin, vorgesehen ist.

Die bereits ausgeführten Beschreibungen bezüglich der Verwendung des Messgeräts, insbesondere die Ausführungen zur Auswertevorrichtung und zum Kernspinresonanz-Sensor, gelten entsprechend natürlich auch für das Messgerät selbst.

In einer Ausführungsform des handgehaltenen Messgeräts ist ferner eine Speichervorrichtung zum Speichern von Messergebnissen und/oder Arbeitsparametern vorgesehen. Diese Speichervorrichtung kann alle Formen an externen und internen elektronischen, insbesondere digitalen Speichern, umfassen, insbesondere auch Speicherchips wie USB-Sticks, Memory-Sticks, Speicherkarten, etc.

Darüber hinaus wird vorgeschlagen, dass die Steuervorrichtung und/oder die Auswertevorrichtung des erfindungsgemäßen Messgeräts eine Datenkommunikationsschnittstelle zur, insbesondere drahtlosen, Kommunikation aufweist, mittels der das Messgerät Messergebnisse und/oder Arbeitsparameter senden und/oder empfangen kann. Bevorzugt verwendet die Datenkommunikationsschnittstelle ein standardisiertes Kommunikationsprotokoll zu einer Übertragung von elektronischen, insbesondere digitalen Daten. Vorteilhaft umfasst die Datenkommunikationsschnittstelle eine drahtlose Schnittstelle, insbesondere beispielsweise eine WLAN-, Bluetooth-, Infrarot-, NFC-, RFID-, GSM-Schnittstelle oder eine andere, einem Fachmann als sinnvoll erscheinende drahtlose Schnittstelle. Alternativ kann die Datenkommunikationsschnittstelle auch einen kabelgebunden Adapter aufweisen, beispielsweise einen USB- oder Mikro-USB-Adapter. Vorteilhaft können mittels der Datenkommunikationsschnittstelle Messergebnisse und/oder Arbeitsparameter von dem Messgerät an ein externes Datengerät, beispielsweise an ein Smartphone, einen Tablet-PC, einen PC, einen Drucker oder weitere einem Fachmann als sinnvoll erscheinende externe Geräte gesendet werden oder von diesen empfangen werden. Mittels der erfindungsgemäßen Ausgestaltung kann vorteilhaft eine Übertragung von Referenzdaten ermöglicht werden, die zu einer weiteren Auswertung von mit dem Messgerät erfassten Messsignalen nutzbar ist. Insbesondere werden die Referenzdaten dabei von einer geräteinternen Referenzdatenbank abgerufen. Ferner können vorteilhaft vielfältige Zusatzfunktionen ermöglicht und eingebunden werden, die insbesondere auch eine direkte Kommunikation mit Smartphones (insbesondere über programmierte Apps) oder ähnlichen portablen Datengeräten erfordern. Diese können beispielsweise automatische Kartierungs-Funktionen, Firmware-Updates, Datennachbearbeitung, Datenaufbereitung, Datenabgleich mit anderen Geräten, etc. umfassen.

Ferner wird ein erfindungsgemäßes Verfahren zur Untersuchung eines Bestandteils eines menschlichen oder tierischen Körpers, insbesondere zur Untersuchung von Gewebe und/oder Körperflüssigkeiten, bevorzugt zur Untersuchung von Blut und/oder Urin, mittels eines handgehaltenen, energieautonomen Messgeräts vorgeschlagen. In einer bevorzugten Ausführungsform des Verfahrens kann das Verfahren insbesondere durch zumindest folgende Schritte gekennzeichnet sein:
i. Spezifizieren von Angaben betreffend einen zu untersuchenden Bestandteil eines menschlichen oder tierischen Körpers unter Verwendung einer Eingabevorrichtung
ii. Kalibrieren des Kernspinresonanz-Sensors unter Verwendung einer Standardprobe, insbesondere unter Verwendung einer geräteintern vorgesehenen Tetramethylsilan-Probe
iii. Anlegen des Messgeräts an den zu untersuchenden Bestandteil eines menschlichen oder tierischen Körpers oder Anlegen des zu untersuchenden Bestandteils eines menschlichen oder tierischen Körpers an das Messgerät
iv. Messung zumindest eines Spektrums und/oder einer Relaxationszeit resultierend aus der Anregung von Kernspins in dem zu untersuchenden Bestandteil eines menschlichen oder tierischen Körpers
v. Auswertung von Messsignalen des Kernspinresonanz-Sensors durch Vergleich der Messsignale mit Referenzdaten einer Referenzdatenbank
vi. Ausgabe der Auswerteergebnisse, insbesondere einer ermittelten Information und/oder einer Abweichung einer ermittelten Information von Referenzdaten einer Referenzdatenbank.

### Zeichnungen

Die Erfindung ist anhand von in den Zeichnungen dargestellten Ausführungsbeispielen in der nachfolgenden Beschreibung näher erläutert. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Gleiche oder ähnliche Bezugszeichen in den Figuren bezeichnen gleiche oder ähnliche Elemente.

Es zeigen:
- Figur 1: ein perspektivische Darstellung einer Ausgestaltung des erfindungsgemäßen mobilen Messgeräts,
- Figur 2: eine Ansicht der ersten Gehäuseseite einer Ausgestaltung des erfindungsgemäßen Messgeräts,
- Figur 3: eine schematische Seitenansicht einer Ausgestaltung des erfindungsgemäßen Messgeräts,
- Figur 4a: eine schematische Schnittdarstellung einer Ausführungsform der den Kernspinresonanz-Sensor bildenden Komponenten sowie der damit erzeugten Magnetfelder,
- Figur 4b: eine schematische Schnittdarstellung einer alternativen Ausführungsform der den Kernspinresonanz-Sensor bildenden Komponenten sowie der damit erzeugten Magnetfelder,
- Figur 5: ein Verfahrensdiagramm einer Ausführungsform des erfindungsgemäßen Verfahrens.

### Beschreibung der Ausführungsbeispiele

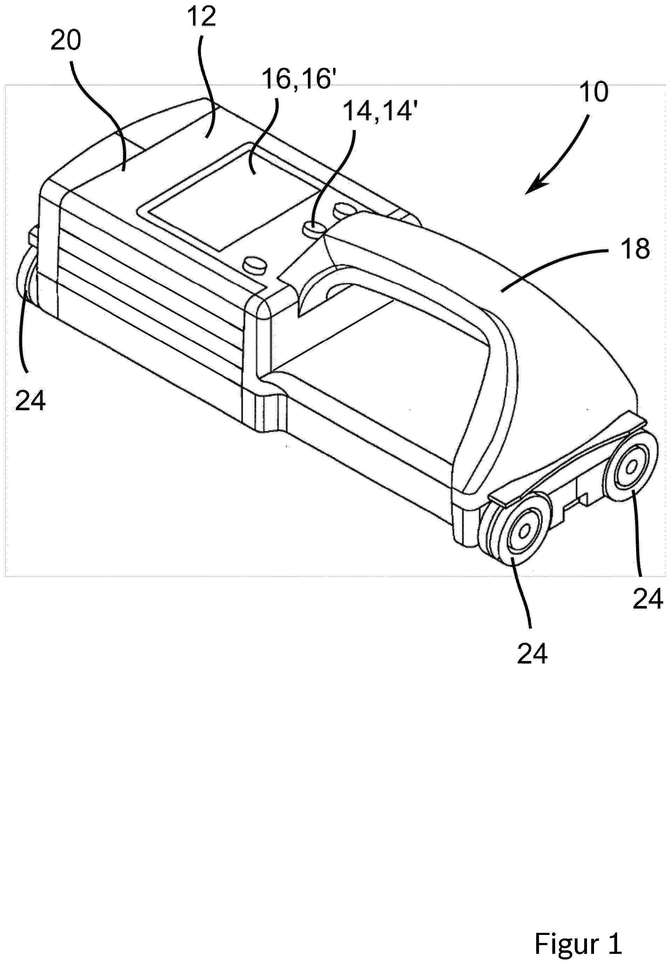

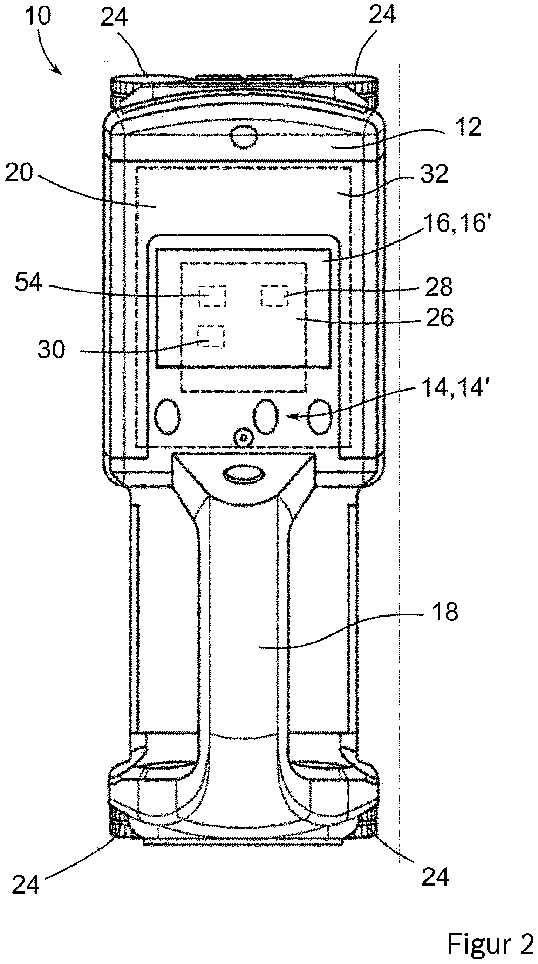

Figur 1 und Figur 2 zeigen zwei Ansichten einer beispielhaften Ausführungsform des erfindungsgemäßen handgehaltenen, energieautonomen Messgeräts 10 in perspektivischer Darstellung bzw. in vereinfachter, schematischer Aufsicht.

Das beispielhaft ausgeführte Messgerät 10 weist ein Gehäuse 12 auf. In dem Gehäuse 12 ist eine Eingabevorrichtung 14 in Form von Betätigungselementen 14', geeignet zum Ein- und Ausschalten des Messgeräts 10, zum Starten und Konfigurieren eines Messvorgangs und zum Eingeben von Arbeitsparametern, untergebracht. Ferner ist in dem Gehäuse 12 eine Ausgabevorrichtung 16 zur Ausgabe von ermittelten Informationen sowie zur Ausgabe von Arbeitsparametern in Form eines Displays 16' vorgesehen. Das Messgerät 10 verfügt zum Transport und zu dessen Führung über einen Handgriff 18. Der Handgriff 18, die Betätigungselemente 14' sowie das Display 16' befinden sich auf einer ersten Gehäuseseite 20 des Messgeräts 10 (auch "Frontseite"), die bei einer Bedienung des Messgeräts typischerweise dem Bediener zugewandt ist.

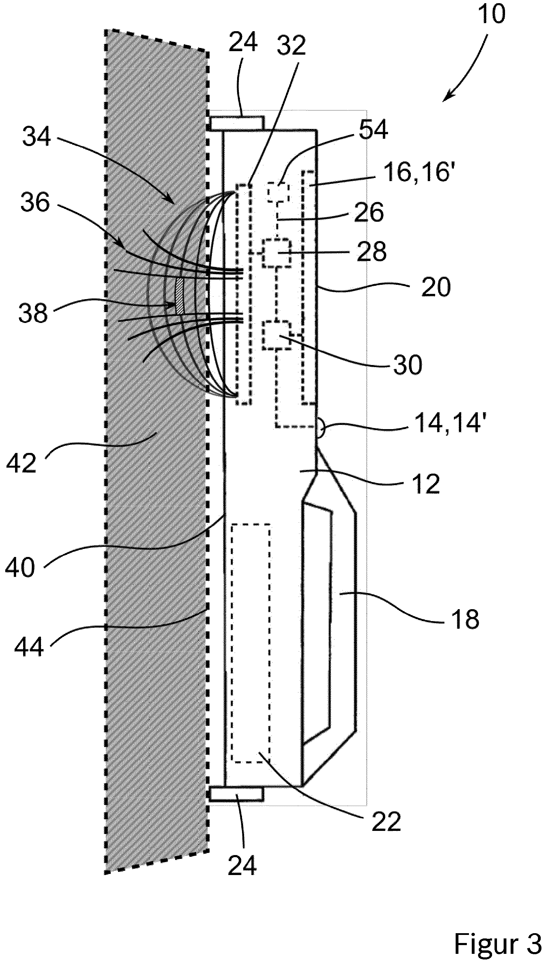

Zur Energieversorgung des Messgerät 10 weist das Messgerät 10 auf der, der ersten Gehäuseseite 20 geräterückseitig gegenüberliegenden, zweiten Gehäuseseite 40 (im Folgenden auch Rückseite des Messgeräts genannt) eine Aussparung auf, die der Aufnahme von stromnetzunabhängigen Energiespeichern 22 in Form von wiederaufladbaren Akkus dient (vgl. Figur 3). Auf Grund des stromnetzunabhängigen Energiespeichers 22 kann das Messgerät 10 zumindest vorübergehend energieautonom, d.h. unabhängig von einem Stromnetz und somit insbesondere auch kabellos, betrieben werden. Das beispielhaft vorgestellte Messgerät 10 besitzt Lithium-Ionen-Akkus, deren hohe Energie- und Leistungsdichte vorteilhaft zur Energieversorgung des Messgeräts 10 geeignet ist. In einer alternativen Ausführungsform kann der Energiespeicher 22 auch im Handgriff 18 des Messgeräts 10 untergebracht sein. Vorzugsweise weist die Energieversorgungsvorrichtung eine lösbare Formschluss- und/oder Kraftschlussverbindungsschnittstelle auf, sodass der Energiespeicher 22 (im Allgemeinen auch mehrere) abnehmbar und austauschbar anordenbar ist. Darüber hinaus lässt sich der Energiespeicher 22 in und/oder außerhalb des Messgeräts 10 mit Energie aus einem Stromnetz versorgen und laden.

Das Messgerät 10 weist eine Positionsbestimmungsvorrichtung in Form von vier Rädern 24 auf, mittels der das Messgerät 10 entlang der Oberfläche 44 eines Bestandteils 42 eines menschlichen oder tierischen Körpers verfahren werden kann (vgl. insbesondere Figur 3). Sensoren, die auf eine Drehung der Räder 24 sensitiv sind, erfassen eine Bewegung des Messgeräts 10 und erlauben somit Messergebnisse in Bezug zu einer Position des Messgeräts 10, insbesondere bezogen auf den Bestandteil 42, zu setzen. Nach Aufsetzen des handgehaltenen Messgeräts 10 auf der Oberfläche 44 eines zu untersuchenden Bestandteils 42 eines menschlichen oder tierischen Körpers wird die Positionsänderung des Messgeräts 10 in Folge eines Verfahrens des Messgeräts 10 auf dem Bestandteil 42 eines menschlichen oder tierischen Körpers ermittelt. Diese Positionsdaten werden zur weiteren Auswertung an eine Auswertevorrichtung 30 weitergegeben.

Auf einem Trägerelement 26, insbesondere einer Systemplatine oder Leiterplatte innerhalb des Gehäuses 12, sind weitere Komponenten des Messgeräts 10, insbesondere ein Kernspinresonanz-Sensor 32, eine Steuervorrichtung 28 zur Steuerung des Messgeräts 10, eine Auswertevorrichtung 30 zur Auswertung von von dem Kernspinresonanz-Sensor 32 gelieferten Messsignalen, sowie eine mit der Steuer- und/oder Auswertevorrichtung verbundene Datenkommunikationsschnittstelle 54, untergebracht (siehe insbesondere Figur 2).

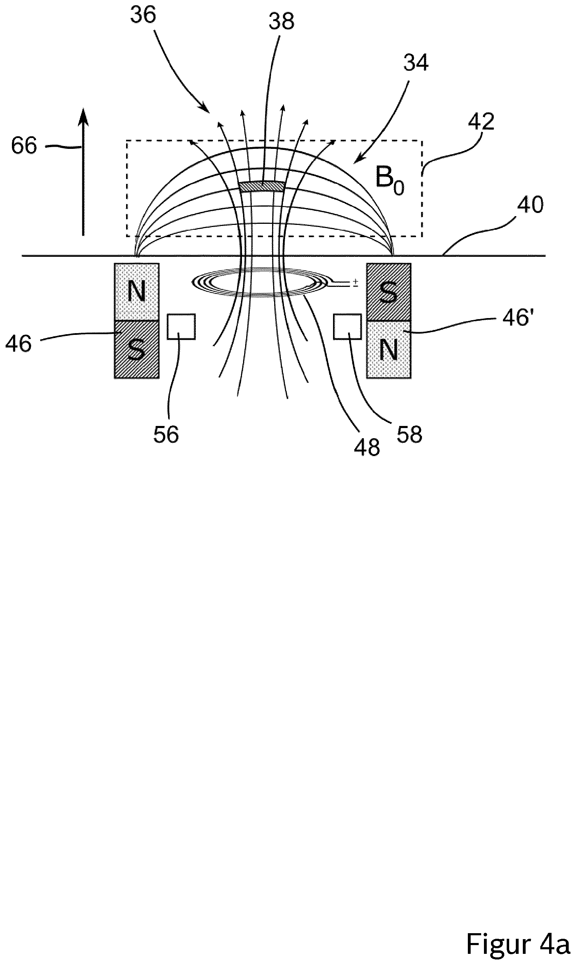

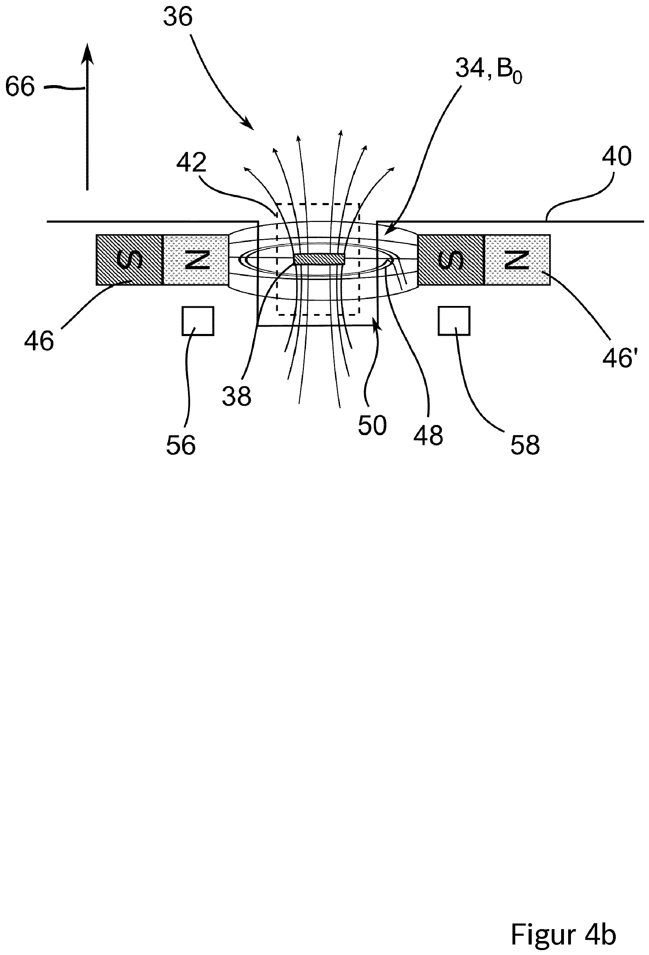

Der Kernspinresonanz-Sensor 32, der im Detail in der Beschreibung zu den Figuren 4a und 4b erläutert wird, ist zur Anregung einer Kernspinresonanz in Atomkernen des Materials des Bestandteils 42 eines menschlichen oder tierischen Körpers vorgesehen. Erfindungsgemäß wird das gemessene Resonanzsignal, insbesondere ein Spektrum, Relaxationskurven und/oder Relaxationszeiten, zumindest zur zerstörungsfreien Unterscheidung des Bestandteils 42 eines menschlichen oder tierischen Körpers verwendet. Auf diese Weise können Informationen ermittelt werden, die unter anderem den Zustand, eine Behandlungsfälligkeit, eine Behandlungsfähigkeit, eine Zusammensetzung des Bestandteils 42 eines menschlichen oder tierischen Körpers, das Vorhandensein von Stoffkomponenten sowie deren Konzentration oder dergleichen betreffen.

Die Steuervorrichtung 28 weist eine Steuerelektronik umfassend Mittel zur Kommunikation mit den anderen Komponenten des Messgeräts 10 auf, beispielsweise Mittel zur Steuerung und Regelung des Kernspinresonanz-Sensors 32, der Auswertevorrichtung 30 und dergleichen. Die Steuervorrichtung 28 umfasst insbesondere eine Einheit mit einer Prozessoreinheit, einer Speichereinheit und einem in der Speichereinheit gespeicherten Betriebsprogramm. Die Steuervorrichtung 28 ist dazu vorgesehen, zumindest ein Betriebsfunktionsparameter des Messgeräts 10 in Abhängigkeit von zumindest einer Eingabe durch den Bediener, durch die Auswertevorrichtung 30 und/oder durch die Datenkommunikationsschnittstelle 54 einzustellen.

Die Auswertevorrichtung 30 zur Auswertung von von dem Kernspinresonanz-Sensor 32 gelieferten Messsignalen weist insbesondere einen Informationseingang, eine Informationsverarbeitung und einen Informationsausgang auf (nicht näher dargestellt). Vorteilhaft besteht die Auswertevorrichtung 30 zumindest aus einem Prozessor, einem Speicher mit einem darauf gespeicherten und ausführbaren Betriebsprogramm und erlaubt, zumindest ein Messsignal des Kernspinresonanz-Sensors 32 auszuwerten und somit Informationen betreffend den Zustand, eine Behandlungsfälligkeit, eine Behandlungsfähigkeit, eine Zusammensetzung des Bestandteils 42 eines menschlichen oder tierischen Körpers, das Vorhandensein von Stoffkomponenten sowie deren Konzentration oder dergleichen zu bestimmen. Ferner weist die Auswertevorrichtung 30 gespeicherte Korrektur- und/oder Kalibriertabellen auf, die es erlauben, die Auswerteergebnisse zu interpretieren, umzurechnen, zu inter- und/oder extrapolieren sowie das Messgerät 10, insbesondere die Auswerteroutinen, hinsichtlich eines Bestandteils 42 eines menschlichen oder tierischen Körpers zu kalibrieren. Die Auswerteergebnisse werden von der Auswertevorrichtung 30 zur weiteren Verwendung über die Steuervorrichtung 28 entweder direkt an einen Bediener des Messgeräts 10 oder zur Versendung der Daten an die Datenkommunikationsschnittstelle 54 ausgegeben. Insbesondere können die Auswerteergebnisse und/oder Messsignale unter Verwendung der Datenkommunikationsschnittstelle 54 mit in einer Referenzdatenbank gespeicherten Referenzdaten verglichen werden.

In Figur 3 ist die Ausführungsform des handgehaltenen Messgeräts 10 der Figuren 1 und 2 in einer vereinfachten schematischen Seitenansicht dargestellt. Der Kernspinresonanz-Sensor 32 umfasst zwei Vorrichtungen zur Erzeugung von Magnetfeldern, insbesondere eine Permanentmagnetanordnung 46,46' (vgl. Figur 4a, 4b), die ein erstes Magnetfeld 34 erzeugt (B₀), sowie eine Hochfrequenzspule 48 (vgl. Figur 4a, 4b), die ein zweites Magnetfeld 36 erzeugt. Der Kernspinresonanz-Sensor 32 ist derart konfiguriert, dass das erste Magnetfeld 34 im Wesentlichen parallel zu der zweiten Gehäuseseite 40 ausgerichtet ist, während das zweite Magnetfeld 36 im Wesentlichen senkrecht zu den Magnetfeldlinien des ersten Magnetfeldes 34 ausgerichtet ist. Die beiden Magnetfelder überlagern sich in einem ausgedehnten Bereich, in dem sich der sensitive Bereich 38 des Kernspinresonanz-Sensors 32 als insbesondere schichtförmiger Bereich befindet. Das handgehaltene Messgerät 10 wird mit der zweiten Gehäuseseite 40 in unmittelbarer Nähe an einen zu untersuchenden Bestandteil 42 eines menschlichen oder tierischen Körpers derart positioniert, dass der Abstand zwischen der zweiten Gehäuseseite 40 und der Oberfläche 44 des Bestandteils 42 eines menschlichen oder tierischen Körpers minimiert ist. Auf diese Weise wird erreicht, dass die Magnetfelder 34,36 in den Bestandteil 42 eines menschlichen oder tierischen Körpers eindringen und der sensitive Bereich 38 in dem Bestandteil 42 eines menschlichen oder tierischen Körpers zu liegen kommt.

Durch Variation des durch die zweite Vorrichtung erzeugten zweiten Magnetfeldes 36, d.h., insbesondere durch Variation der Hochfrequenzspule 48 und/oder Variation der Frequenz und/oder Variation des Stroms und/oder Variation der Spannung in der Hochfrequenzspule 48, ist es möglich, den sensitiven Bereich 38 in seinem Abstand zu der zweiten Gehäuseseite 40 (in Richtung 66 in den Bestandteil 42 eines menschlichen oder tierischen Körpers hinein) zu verändern und somit den Abstand des sensitiven Bereichs 38 im Bestandteil 42 eines menschlichen oder tierischen Körpers zu dessen Oberfläche 44 zu modifizieren. Alternativ und/oder zusätzlich kann der Kernspinresonanz-Sensor 32 im Gehäuse 12 des Messgeräts 10 derart umpositioniert werden, dass der Abstand des Kernspinresonanz-Sensors 32 zur zweiten Gehäuseseite 40 verändert wird und folglich auch der Abstand des sensitiven Bereichs 38 im Bestandteil 42 zu dessen Oberfläche 44. Besonders vorteilhaft lassen sich auf diese Weise Tiefenprofile der auszuwertenden Informationen erstellen. Beispielsweise ist es möglich, über ein Tiefenprofil eines Feuchteverlaufs in einem Bestandteil 42 eines menschlichen oder tierischen Körpers eine Aussage über den Fortschritt eines Zersetzungsprozesses zu treffen.

In Figur 4a sind in schematischer Schnittdarstellung eines Details des Ausführungsbeispiels des Messgeräts 10 der Figuren 1 bis 3 der Kernspinresonanz-Sensor 32 zusammen mit einem zu untersuchenden Bestandteil 42 eines menschlichen oder tierischen Körpers dargestellt. Zwei senkrecht zur zweiten Gehäuseseite 40 und antiparallel zueinander angeordnete Permanentmagnete 46, 46' erzeugen ein erstes, insbesondere statisches, Magnetfeld 34, das im Wesentlichen parallel zur Oberfläche der zweiten Gehäuseseite 40 verläuft. Dieses zur Ausrichtung der Kernspins der in dem Bestandteil 42 eines menschlichen oder tierischen Körpers vorhandenen Atomkerne vorgesehene erste Magnetfeld 34 weist beispielhaft insbesondere eine Magnetfeldstärke von 0.5 Tesla auf, wobei die Permanentmagnete 46,46' aus einer Neodym-Eisen-Bor-Legierung hergestellt sind. In einer alternativen Ausführungsform kann das Magnetfeld 34 auch mittels eines Elektromagneten erzeugt werden. Die zweite Vorrichtung zur Erzeugung des zweiten Magnetfeldes wird in diesem Ausführungsbeispiel durch eine Hochfrequenzspule 48 gebildet. Sobald durch diese Spule Strom fließt, wird ein elektromagnetisches Feld, insbesondere das zweite Magnetfeld 36, induziert. Die beiden Magnetfelder überlagern sich in einem Bereich, der im Wesentlichen außerhalb des Gehäuses 12 des Messgeräts 10 liegt. Der sensitive Bereich 38 des Kernspinresonanz-Sensors 32 liegt ebenfalls in dem Überlagerungsfeld der Magnetfelder 34 und 36. In Abhängigkeit der Frequenz des eingestrahlten elektromagnetischen Felds 36 und der statischen Magnetfeldstärke des ersten Magnetfelds 34 wird der sensitive Bereich im Idealfall durch eine Fläche definiert, auf der die Magnetfeldstärke des ersten Magnetfelds 34 konstant ist und insbesondere einen definierten Betrag aufweist. In Realität ist die Fläche auf Grund nicht exakter Frequenzen tatsächlich schichtförmig. Da die Magnetfeldlinien 34 nicht exakt parallel zur zweiten Gehäuseseite 40 verlaufen, ist somit auch der sensitive Bereich 38 folglich entsprechend der Magnetfeldlinien gekrümmt. Die Krümmung und Ausformung des ersten Magnetfelds 34 und damit des sensitiven Bereichs 38 kann unter Verwendung weiterer Mittel, beispielsweise einer Shim-Spule 56 und einer magnetischen Schirmung 58, beeinflusst und insbesondere homogenisiert werden.

Die Oberfläche 40 des Gehäuses 12 des Messgeräts 10 stellt in diesem Ausführungsbeispiel eine ebene Fläche dar, an der der Bestandteil 42 eines menschlichen oder tierischen Körpers angelegt werden kann (oder umgekehrt).

In Figur 4b sind in schematischer Schnittdarstellung eines Details einer alternativen Ausführungsform des Messgeräts 10 der Kernspinresonanz-Sensor 32 zusammen mit einem zu untersuchenden Bestandteil 42 eines menschlichen oder tierischen Körpers dargestellt. Dabei ist das durch die erste Vorrichtung, hier zwei parallel zur zweiten Gehäuseseite und kollinear angeordnete Permanentmagnete 46,46' (in Nord-Süd/Nord-Süd-Abfolge), erzeugte erste, insbesondere statische, Magnetfeld 34 im Wesentlichen parallel zu einer zweiten Gehäuseseite 40 des Messgeräts 10 und das durch die zweite Vorrichtung, hier eine Hochfrequenzspule 48, erzeugte zweite Magnetfeld 36 im Wesentlichen senkrecht zu dem ersten Magnetfeld 34 ausgerichtet. Zwischen den beiden Permanentmagneten 46,46' befindet sich eine Hochfrequenzspule 48, deren Wicklungsebene kollinear zur Erstreckungsrichtung der Permanentmagnete 46,46' und parallel zur zweiten Gehäuseseite 40 liegt. Diese Anordnung ist in unmittelbarer Nähe zur zweiten Gehäuseseite 40 positioniert. Sobald durch diese Spule Strom fließt, wird ein elektromagnetisches Feld, insbesondere das zweite Magnetfeld 36, induziert. Die beiden Magnetfelder überlagern sich in einem Bereich, der im Wesentlichen außerhalb des Gehäuses 12 des Messgeräts 10 liegt. Der sensitive Bereich 38 des Kernspinresonanz-Sensors 32 liegt ebenfalls in dem Überlagerungsfeld der Magnetfelder 34 und 36. In Abhängigkeit der Frequenz des eingestrahlten elektromagnetischen Felds 36 und der statischen Magnetfeldstärke des ersten Magnetfelds 34 wird der sensitive Bereich im Idealfall durch eine Fläche definiert, auf der die Magnetfeldstärke des ersten Magnetfelds 34 konstant ist und insbesondere einen definierten Betrag aufweist. In Realität ist die Fläche auf Grund nicht exakter Frequenzen tatsächlich schichtförmig. Da die Magnetfeldlinien 34 nicht exakt parallel zur zweiten Gehäuseseite 40 verlaufen, ist somit auch der sensitive Bereich 38 folglich entsprechend der Magnetfeldlinien gekrümmt. Die Krümmung und Ausformung des ersten Magnetfelds 34 und damit des sensitiven Bereichs 38 kann unter Verwendung weiterer Mittel, beispielsweise einer Shim-Spule 56 und einer magnetischen Schirmung 58, beeinflusst und insbesondere homogenisiert werden.

Die Oberfläche 40 des Gehäuses 12 des Messgeräts 10 stellt in diesem Ausführungsbeispiel allerdings keine ebene Fläche wie in den Figuren 3 und 4a dar, sondern weist eine speziell zur Aufnahme eines zu untersuchenden Bestandteils 42 eines menschlichen oder tierischen Körpers ausgeformte Vertiefung 50 auf. Die Vertiefung 50 sowie die Anordnung des Kernspinresonanz-Sensors 30 sind derart aufeinander abgestimmt, dass der Bestandteil 42 eines menschlichen oder tierischen Körpers, beispielsweise eine in einem Reagenzglas befindliche Blut- oder Urinprobe, in die Vertiefung 50 eingeführt wird. Der sensitive Bereich 38 des Kernspinresonanz-Sensors 32 kommt dabei unmittelbar in dem Bestandteil 42 des menschlichen oder tierischen Körpers zu liegen. Ferner kann die Vertiefung auch zur Aufnahme bzw. Einführung eines Körperteils, beispielsweise eines Fingers oder einer Hand, ausgeformt sein.

In einer Ausführungsform ist die Vertiefung 50 als ein zylindrisch umschlossener Hohlraum realisiert, wobei die Vertiefung selbst von einem zylindrischen Permanentmagneten, beispielsweise in Halbach-Anordnung, umgeben ist. Somit umschließt der Permanentmagnet den in das Messgerät 10 eingeführten Bestandteil 42 eines menschlichen oder tierischen Körpers auf der gesamten Länge dessen Umfangs. Ferner ist derart ein besonders homogenes erstes Magnetfeld 34 (B₀) erzeugbar. Alternativ oder zusätzlich kann das erste Magnetfeld 34 in diesem Ausführungsbeispiel auch mit einer oder mehreren runden Spulen oder runden Magneten erzeugt werden.

Weitere Ausformungen des Messgeräts 10, insbesondere dessen Kernspinresonanz-Sensors 32, die auf einen bestimmten Verwendungsort und/oder eine bestimmte Verwendungsart ausgelegt sind, sind denkbar. Beispielsweise kann zur Messung an einem Ohrläppchen ein U- oder C-förmiger Permanentmagnet verwendet werden. Je weniger tief der sensitive Bereich 38 des Kernspinresonanz-Sensors 32 in einen Bestandteil 42 eines menschlichen oder tierischen Körpers eindringen muss, desto günstiger und kleiner kann das Gerät realisiert werden, da die erforderliche Magnetfeldstärke B₀ drastisch mit sinkender Eindringtiefe des Magnetfelds 34 abnimmt.

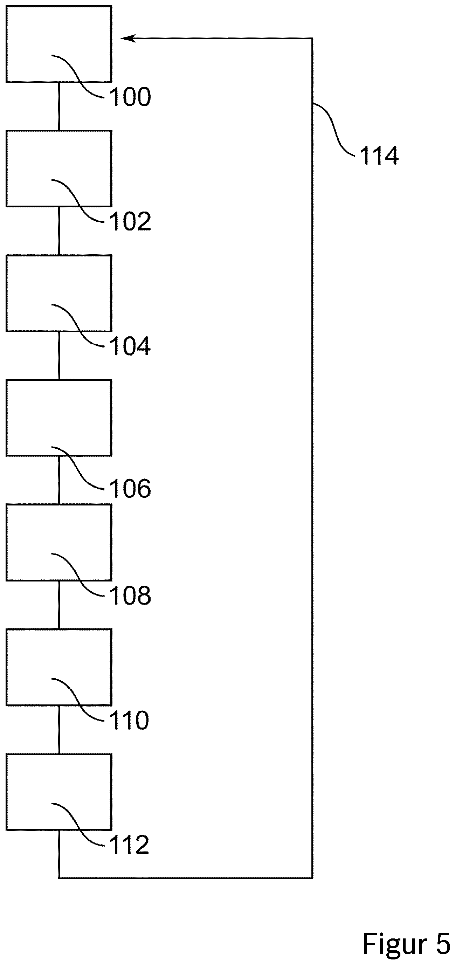

In der Figur 5 ist ein Verfahrensdiagramm gezeigt, dass ein Ausführungsbeispiel des erfindungsgemäßen Verfahrens zur Untersuchung eines Bestandteils 42 eines menschlichen oder tierischen Körpers mittels eines handgehaltenen, energieautonomen Messgeräts 10 darstellt. Als Anwendungsszenario ist beispielsweise denkbar, dass ein Bediener des Messgeräts 10 an Diabetes erkrankt ist und eine regelmäßige Blutzuckermessung durchführen muss, aus der er ableiten kann, ob er eine Insulininjektion vornehmen muss (im Falle erhöhter Glukosekonzentration im Blut). Dass Messgerät 10 erinnert unter Verwendung der Ausgabevorrichtung 16, beispielsweise durch einen Vibrationsalarm, an die Durchführung einer Messung.

In einem ersten Verfahrensschritt 100 wird das Messgerät 10 dann von dem Bediener eingeschaltet und befindet sich nach einer kurzen Hochlaufzeit in einem Leerlaufmodus. Anschließend werden in Verfahrensschritt 102 unter Verwendung der Eingabevorrichtung 14 Angaben betreffend den zu untersuchenden Bestandteil 42 eines menschlichen oder tierischen Körpers spezifiziert, hier beispielsweise die Auswahl von "Finger" und des Bedienerprofils mit Angaben zum Gewicht und dergleichen. In Verfahrensschritt 104 findet eine Kalibrierung des Kernspinresonanz-Sensors 32 unter Verwendung einer geräteintern vorgesehenen Tetramethylsilan-Probe statt, in deren Anschluss das Messgerät 10 für die Untersuchung des Bestandteils 42 eines menschlichen oder tierischen Körpers einsatzbereit ist. Zur Messung eines Kernspinresonanzsignals in dem Bestandteil 42, hier des Fingers, wird der Finger in Verfahrensschritt 106 in die Vertiefung 50 des Messgeräts 10 in unmittelbarer Nähe zu dem Kernspinresonanz-Sensor 32 eingeführt - hier entsprechend der geometrischen Ausführung des Messgeräts 10 nach dem Ausführungsbeispiel aus Figur 4b. Dabei dringen die durch den Kernspinresonanz-Sensor 32 erzeugten Magnetfelder 34,36 durch die zweite Gehäuseseite 40 aus dem Messgerät 10 aus und in den Finger (Bestandteil 42) des Bedieners ein, wobei der sensitive Bereich 38 in dem Finger zu liegen kommt (siehe insbesondere Figur 4b). Magnetfeldänderungen in Folge eines Kernspinresonanzeffekts der in dem Finger angeregten Kernspins der Atomkerne, d.h. verursacht durch Absorption und/oder Emission elektromagnetischer Felder durch die Atomkerne einhergehend mit einer Änderung deren Energiezustände, wird mittels der Hochfrequenzspule 48 des Kernspinresonanz-Sensors 32 detektiert (Verfahrensschritt 108). Dieses Messsignal, insbesondere ein Messsignal repräsentierend ein Spektrum und/oder Relaxationskurven, wird an die Auswertevorrichtung 30 weitergeleitet, von der es mittels Auswerteroutinen aufbereitet, insbesondere gefiltert und/oder geglättet wird. Anschließend erfolgt eine Auswertung des Messsignals des Kernspinresonanz-Sensors durch Vergleich des Messsignals mit Referenzdaten einer Referenzdatenbank (Verfahrensschritt 110). Dabei werden die gemessenen Spektren und/oder Relaxationskurven und/oder Relaxationszeiten mit Referenzspektren bzw. Referenzrelaxationskurven bzw. Referenzrelaxationszeiten abgeglichen. Die Erkennung von Übereinstimmungen und/oder Abweichungen des Messsignals mit bzw. von den Referenzdaten erlaubt in diesem Verfahrensschritt die schnelle und präzise Auswertung des Messsignals, bei der Informationen betreffend den untersuchten Bestandteil 42 des menschlichen oder tierischen Körpers, hier des Fingers, erhalten und aufbereitet werden. Die Auswerteergebnisse, insbesondere die ermittelte Information und/oder eine Abweichung der ermittelten Information von Referenzdaten der Referenzdatenbank, werden anschließend an die Ausgabevorrichtung 16 weitergeleitet (Verfahrensschritt 112). Das ausgewertete Messergebnis, d.h. die Information betreffend den untersuchte Bestandteil 42 des menschlichen oder tierischen Körpers, wird dem Bediener auf dem Display 16' dargestellt und kann zusätzlich über die Datenkommunikationsschnittstelle 54 an ein weiteres Datenverarbeitungsgerät gesendet werden. Die Ausgabe auf dem Display 16' kann grafisch, numerisch und/oder alphanumerisch, beispielsweise in Form eines Messwerts, einer Messkurve, eines Signalverlaufs, eines Zeitverlauf, als Bilddaten oder in einer Gradientendarstellung sowie in einer Kombination derer erfolgen. Alternativ oder zusätzlich ist eine Darstellung mittels einer Signalanzeige möglich, insbesondere beispielsweise einer Leuchtdiode, die beispielsweise über eine Farbcodierung (z.B. rot, gelb, grün) eine Zielgröße, hier den Blutzuckergehalt und/oder die Notwendigkeit einer Insulininjektion, bewertet. Das Verfahren wiederholt sich, insbesondere bei einer weiteren Untersuchung des Bestandteils 42 eines menschlichen oder tierischen Körpers, angedeutet durch den Pfeil 114.

Auf diese Weise kann in diesem Anwendungsszenario der Bediener unter Verwendung des Messgeräts 10 nicht-invasiv eine Blutzuckerbestimmung durchführen. Ferner könnten mit mehreren Messungen in kurzer Folge Prognosen betreffend den Zeitpunkt einer nächsten Messung erstellt werden. Ferner kann eine eingebaute Fehlmessungserkennung eine Warnung ausgeben, sofern eine Messung fehlerhaft ist oder sein könnte, beispielsweise in Folge eines während der Messung bewegten Fingers (beispielsweise kapazitiv detektiert oder mittels einer Lichtschranke).

## Patentansprüche

1. Verwendung eines handgehaltenen, energieautonomen Messgeräts (10) mit einem Gehäuse (12), in dem zumindest ein Kernspinresonanz-Sensor (32), eine Steuervorrichtung (28) zur Steuerung des Messgeräts (10), eine Auswertevorrichtung (30) zur Auswertung eines von dem Kernspinresonanz-Sensor (32) gelieferten Messsignals, eine Ausgabevorrichtung (16, 16') zur Ausgabe von ermittelten Informationen sowie eine Energieversorgungsvorrichtung (22) des Messgeräts (10) in Form einer Batterie, insbesondere einer wiederaufladbaren Batterie, vorgesehen sind, zur Untersuchung eines Bestandteils (42) eines menschlichen oder tierischen Körpers, insbesondere zur Untersuchung von Gewebe und/oder Körperflüssigkeiten, bevorzugt zur Untersuchung von Blut und/oder Urin.

2. Verwendung des Messgeräts (10) nach Anspruch 1, wobei mittels der Auswertevorrichtung (30) des Messgeräts (10) das von dem Kernspinresonanz-Sensor (32) gelieferte Messsignal, insbesondere ein Spektrum und/oder eine Relaxationszeit des Messsignals resultierend aus der Anregung von Kernspins in dem zu untersuchenden Bestandteil (42) eines menschlichen oder tierischen Körpers durch den Kernspinresonanz-Sensor (32), ausgewertet wird.

3. Verwendung des Messgeräts (10) nach einem der Ansprüche 1-2, wobei mittels der Auswertevorrichtung (30) des Messgeräts (10) eine ermittelte Information, insbesondere ein Spektrum und/oder eine Relaxationszeit, mit Referenzdaten einer Referenzdatenbank verglichen wird.

4. Verwendung des Messgeräts nach einem der Ansprüche 1-3, wobei mittels einer Ausgabevorrichtung (16, 16') des Messgeräts (10), insbesondere mittels eines Displays (16'), eine ermittelte Information, insbesondere ein Spektrum und/oder eine Relaxationszeit, und/oder eine Abweichung einer ermittelten Information von Referenzdaten einer Referenzdatenbank ausgegeben werden/wird, insbesondere dargestellt werden/wird.

5. Verwendung des Messgeräts (10) nach einem der Ansprüche 1-4, wobei mittels des Messgeräts (10) der Bestandteil (42) eines menschlichen oder tierischen Körpers ortsaufgelöst, insbesondere positionsaufgelöst und/oder tiefenaufgelöst, untersucht wird.

6. Verwendung des Messgerät (10) nach einem der Ansprüche 1-5, wobei mittels einer Eingabevorrichtung (14, 14') Angaben zu dem zu untersuchenden Bestandteil (42) des menschlichen oder tierischen Körpers durch Benutzereingaben spezifiziert und dem Messgerät (10) zur Verfügung gestellt werden.

7. Verwendung des Messgeräts (10) nach einem der Ansprüche 1-6, wobei vor Untersuchung des Bestandteils (42) eines menschlichen oder tierischen Körpers unter Verwendung einer geräteintern vorgesehenen Standardprobe, insbesondere unter Verwendung einer geräteintern vorgesehenen Tetramethylsilan-Probe, eine Kalibrierung des Messgeräts (10) durchführt wird.

8. Handgehaltenes, energieautonomes Messgerät (10) zur Verwendung nach einem der Ansprüche 1-7 umfassend ein Gehäuse (12), in dem zumindest
• ein Kernspinresonanz-Sensor (32),
• eine Steuervorrichtung (28) zur Steuerung des Messgeräts (10),
• eine Auswertevorrichtung (30) zur Auswertung eines von dem Kernspinresonanz-Sensor (32) gelieferten Messsignals,
• eine Ausgabevorrichtung (16, 16') zur Ausgabe von ermittelten Informationen sowie
• eine Energieversorgungsvorrichtung (22) in Form einer Batterie, insbesondere einer wiederaufladbaren Batterie
vorgesehen sind, **dadurch gekennzeichnet, dass** das Messgerät (10), insbesondere der Kernspinresonanz-Sensor (32) und/oder die Auswertevorrichtung (30), zur Untersuchung eines Bestandteils (42) eines menschlichen oder tierischen Körpers, insbesondere zur Untersuchung von Gewebe und/oder Körperflüssigkeiten, bevorzugt zur Untersuchung von Blut und/oder Urin, vorgesehen ist.

9. Verfahren zur Untersuchung eines Bestandteils (42) eines menschlichen oder tierischen Körpers, insbesondere zur Untersuchung von Gewebe und/oder Körperflüssigkeiten, bevorzugt zur Untersuchung von Blut und/oder Urin, mittels eines handgehaltenen, energieautonomen Messgerät (10) nach Anspruch 8, **gekennzeichnet durch** zumindest folgende Schritte:
i. Spezifizieren von Angaben betreffend einen zu untersuchenden Bestandteil (42) eines menschlichen oder tierischen Körpers unter Verwendung einer Eingabevorrichtung (14, 14') (Verfahrensschritt 102)
ii. Kalibrieren des Kernspinresonanz-Sensors (32) unter Verwendung einer Standardprobe, insbesondere unter Verwendung einer geräteintern vorgesehenen Tetramethylsilan-Probe (Verfahrensschritt 104)
iii. Anlegen des Messgeräts (10) an den zu untersuchenden Bestandteil (42) eines menschlichen oder tierischen Körpers oder Anlegen des Bestandteils (42) eines menschlichen oder tierischen Körpers an das Messgerät (10) (Verfahrensschritt 106)
iv. Messung zumindest eines Spektrums und/oder einer Relaxationszeit resultierend aus der Anregung von Kernspins in dem zu untersuchenden Bestandteil (42) eines menschlichen oder tierischen Körpers (Verfahrensschritt 108)
v. Auswertung von Messsignalen des Kernspinresonanz-Sensors (32) durch Vergleich der Messsignale mit Referenzdaten einer Referenzdatenbank (Verfahrensschritt 110)
vi. Ausgabe der Auswerteergebnisse, insbesondere einer ermittelten Information und/oder einer Abweichung einer ermittelten Information von Referenzdaten einer Referenzdatenbank (Verfahrensschritt 112)
